# EUROPÄISCHE PATENTANMELDUNG

(11) **EP 0 807 437 A1**
(43) Veröffentlichungstag der Anmeldung: **19.11.1997**
(21) Anmeldenummer: 97107625.2
(22) Anmeldetag: 09.05.1997
(51) Int. Cl.: A61K 31/78

(54) **Polyacrylsäure und Polymethacrylsäure enthaltende Zusammensetzungen mit antiadhäsiven Eigenschaften**

(30) Priorität: 13.05.1996 DE 19619238
(71) Anmelder: HOECHST AKTIENGESELLSCHAFT, 65929 Frankfurt am Main (DE)
(72) Erfinder: Toepfer, Alexander, Dr., 65830 Kriftel (DE); Kretzschmar, Gerhard, Dr., 65760 Eschborn (DE); Schmidt, Wolfgang, Dr., 65929 Frankfurt (DE); Bartnik, Eckart, Dr., 65205 Wiesbaden-Delkenheim (DE); Hüls, Christoph, Dr., 55263 Wackernheim (DE); Seiffge, Dirk, Dr., 55246 Mainz-Kostheim (DE)

(57) **Zusammenfassung**

Die vorliegende Erfindung betrifft Polyacrylsäuren und Polymethacrylsäuren verschiedener molarer Massen zur Anwendung als Arzneimittel und im besonderen zur Anwendung bei der Therapie und Prophylaxe von Erkrankungen, die mit einer übermäßigen, durch Selektinrezeptoren vermittelten Zelladhäsion in dem von der Krankheit betroffenen Gewebe einhergehen, beispielsweise Herz-Kreislauf-Erkrankungen und Rheuma.

## Beschreibung

Die vorliegende Erfindung betrifft Polyacrylsäuren und Polymethacrylsäuren verschiedener molarer Massen zur Anwendung als Arzneimittel und im besonderen zur Anwendung bei der Therapie und Prophylaxe von Erkrankungen, die mit einer übermäßigen, durch Selektinrezeptoren vermittelten Zelladhäsion in dem von der Krankheit betroffenen Gewebe einhergehen.

Die Zirkulation von Blutzellen wie z.B Leukozyten, Neutrophilen, Granulozyten und Monozyten ist auf molekularer Ebene ein vielstufiger, sehr komplexer Prozess, der nur in Teilschritten bekannt ist (Review: T.A.Springer, Cell 76, 301-314, 1994).

Jüngste Forschungsergebnisse zeigten, daß die in der Immunüberwachung entscheidende Rezirkulation der Lymphozyten sowie die Lokalisierung von Neutrophilen und Monozyten an Entzündungsherden sehr ähnlichen molekularen Mechanismen gehorchen. So kommt es bei akuten und chronischen Entzündungsprozessen zur Adhäsion der Leukozyten an Endothelzellen und Auswanderung in den Entzündungsherd und in die sekundären lymphatischen Organe.

An diesem Vorgang sind zahlreiche spezifische Signalmoleküle wie z.B. Interleukine, Leukotriene und Tumornekrosefaktor (TNF), deren G-Protein gekoppelte Rezeptoren und insbesondere gewebespezifische Zelladhäsionsmoleküle beteiligt, die eine genau kontrollierte Erkennung der Immun- und Endothelzellen gewährleisten. Zu den wichtigsten hierbei beteiligten Adhäsionsmolekülen, die im folgenden als Rezeptoren bezeichnet werden sollen, gehören die Selektine (E-, P- und L-Selektine), Integrine und die Mitglieder der Immunglobulin-Superfamilie.

Die drei Selektinrezeptoren bestimmen die Anfangsphase der Leukozytenadhäsion. E-Selektin wird auf Endothelzellen einige Stunden nach der Stimulierung beispielsweise durch Interleukin-1 (IL-1β) oder Tumornekrosefaktor (TNF-α) exprimiert, während P-Selektin in Blutplättchen und Endothelzellen gespeichert wird und nach Stimulierung durch Thrombin, Peroxidradikale oder Substanz P u.a. auf den Zelloberflächen präsentiert wird. L-Selektin wird dauerhaft auf Leukozyten exprimiert, wird im Verlauf der Entzündung jedoch rasch wieder von den Leukozyten abgespalten.

Die in der Anfangsphase entzündlicher Prozesse durch Selektin-Rezeptoren vermittelte Adhäsion von Leukozyten an Endothelzellen ist eine natürliche und notwendige Immunantwort auf diverse Entzündungsreize und Verletzungen des vasculären Gewebes.
Der Verlauf einer Reihe von akuten und chronischen Erkrankungen wird jedoch durch die übermäßige Adhäsion von Leukozyten und deren Infiltration in das betroffene Gewebe sowie durch die Schädigung gesunden Gewebes im Sinne einer Autoimmunreaktion ungünstig beeinflußt. Dazu zählen beispielsweise Rheuma, Reperfusionsverletzungen wie myokardiale Ischämie/Infarkt (MI), akute Lungenentzündung nach operativem Eingriff, traumatischer Schock und Schlaganfall, Psoriasis, Dermatitis, ARDS (Atemnotsyndrom bei Erwachsenen) sowie die nach chirurgischen Eingriffen (Beispiel Angioplastie und By-Pass Operationen) auftretende Restenose.

Der natürliche Ligand wurde schon erfolgreich in Tierversuchen bei P-Selektin abhängigen Lungenverletzungen (M.S.Mulligan et.al., Nature 1993, 364, 149) und bei myokardialen Reperfusionsverletzungen (M.Buerke et.al., J.Clin.Invest. 1994, 93, 1140) verwendet.

Beim Screening nach biologisch aktiven, spezifischen Antagonisten wurde überraschenderweise gefunden, daß (im Handel, beispielsweise bei der Firma Fluka, erhältliche) Polyacrylsäuren und Polymethacrylsäuren in Abhängigkeit von ihrer mittleren Molmasse, die P-selektinvermittelte Zelladhäsion inhibieren.

Demgemäß betrifft die vorliegende Erfindung ein Polymer der Acrylsäure oder der Methacrylsäure oder deren pharmakologisch verträglichen Salze zur Anwendung als Arzneimittel.

Vorzugsweise ist das Polymer dadurch gekennzeichnet, daß es eine Polyacrylsäure, vorzugsweise deren Natriumsalz, ist.

Besonders geeignet zur Anwendung als Arzneimittel sind Polymere der genannten Säuren, deren Molmassen von 900 bis 63 000 g/mol betragen.

Die genannten Polymere sind besonders geeignet zur Herstellung eines Arzneimittels zur Therapie oder Prophylaxe einer Erkrankung, die mit einer übermäßigen, durch Selektinrezeptoren vermittelten Zelladhäsion in dem von der Krankheit betroffenen Gewebe einhergeht, vorzugsweise eine Herz-Kreislauf-Erkrankung oder eine rheumatische Erkrankung.

Die erfindungsgemäßen Arzneimittel werden im allgemeinen intravenös, oral oder parenteral oder als Implantate verabreicht, aber auch eine rektale Anwendung ist prinzipiell möglich. Geeignete feste oder flüssige galenische Zubereitungsformen sind beispielsweise Granulate, Pulver, Tabletten, Dragees, (Mikro-)Kapseln, Zäpfchen, Sirupe, Emulsionen, Suspensionen, Aerosole, Tropfen oder injizierbare Lösungen in Ampullenform sowie Präparate mit protrahierter Wirkstoffe-Freigabe, bei deren Herstellung üblicherweise Trägerstoffe und Zusätze und/oder Hilfsmittel wie Spreng-, Binde-, Überzugs-, Quellungs-, Gleit- oder Schmiermittel, Geschmacksstoffe, Süßungsmittel oder Lösungsvermittler Verwendung finden. Als häufig verwendete Träger- oder Hilfsstoffe seien z.B. Magnesiumcarbonat, Titandioxid, Laktose, Mannit und andere Zucker, Talkum, Milcheiweiß, Gelatine, Stärke, Vitamine, Cellulose und ihre Derivate, tierische und pflanzliche Öle, Polyethylenglykole und Lösungsmittel, wie etwa steriles Wasser, Alkohole, Glycerin und mehrwertige Alkohole genannt.

Vorzugsweise werden die pharmazeutischen Präparate in Dosierungseinheiten hergestellt und verabreicht. Feste Dosierungseinheiten sind Tabletten, Kapseln und Suppositorien.

Für die Behandlung eines Patienten sind je nach Wirksamkeit der Verbindung, Art der Applikation, Art und Schwere der Erkrankung, Alter und Körpergewicht des Patientin, unterschiedliche Tagesdosen notwendig. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleiner Dosierungseinheiten als auch durch Mehrfachgabe unterteilten Dosen in bestimmten Intervallen erfolgen. Die zu verabreichende Tagesdosis kann außerdem von der Anzahl der während des Krankheitsverlaufs exprimierten Rezeptoren abhängig sein. Es ist vorstellbar, daß im Anfangsstadium der Krankheit nur wenige Rezeptoren auf der Zelloberfläche exprimiert werden und demzufolge die zu verabreichende Tagesdosis geringer ist als bei stark erkrankten Patienten.

Die erfindungsgemäßen Arzneimittel werden dadurch hergestellt, daß man die genannten Polymere mit üblichen Träger- sowie gegebenenfalls Zusatz- und /oder Hilfsmittel in die bzw. eine geeignete Darreichungsform bringt.

Die hochmolekularen Polyacrylsäuren und Polymethacrylsäuren stellen sehr potente und spezifische P-Selektin Antagonisten dar. Dies kann anhand der nachfolgend beschriebenen Zelladhäsionsassays nachgewiesen werden.

### Beispiel 1:

Primärassays zur Untersuchung der Wirkung auf die Zellanhaftung an rekombinante, lösliche Selektin-Fusionsproteine.

Um die Wirksamkeit der Polymere auf die Interaktion zwischen den E- und P-Selektinen (alte Nomenklatur ELAM-1 bzw. GMP-140) mit ihren Liganden zu testen, wird ein Assay verwendet, der jeweils nur für eine dieser Interaktionen spezifisch ist. Die Liganden werden in ihrer natürlichen Form als Oberflächenstrukturen auf promyelozytischen HL60 Zellen angeboten. Da HL60 Zellen Liganden und Adhäsionsmoleküle unterschiedlichster Spezifität aufweisen, kann die gewünschte Spezifität des Assays nur über den Bindungspartner erbracht werden. Als Bindungspartner wurden gentechnisch hergestellte lösliche Fusionsproteine aus der jeweils extrazytoplasmatischen Domäne von E- bzw. P-Selektin und der konstanten Region eines humanen Immunglobulins der Subklasse IgG1 verwendet.

### Herstellung von L-Selektin-IgG1

Zur Herstellung von löslichem L-Selektin-IgG1 Fusionsprotein wurde das von Walz et al., 1990 publizierte genetische Konstrukt "ELAM-Rg" verwendet.

Zur Expression wurde die Plasmid DNA in COS-7 Zellen (ATCC) mittels DEAE-Dextran transfiziert (Molekularbiologische Methoden: siehe Ausubel, F.M., Brent, R., Kingston, R.E., Moore, D.D., Seidman, J.G., Struhl, K. und Smith, J.A. 1990. Current Protocols in Molecular Biology, John Wiley, New York). Sieben Tage nach der Transfection wird der Kulturüberstand gewonnen, durch Zentrifugation von Zellen und Zellfragmenten befreit und auf 25 mM Hepes pH 7,0, 0,3 mM PMSF, 0,02 % Natriumazid gebracht und bei +4°C aufgehoben. (Walz, G., Aruffo, A., Kolanus, W., Bevilacqua, M. und Seed, B. 1990. Recognition bei ELAM-1 of the sialyl-Lex determinant on myeloid and tumor cells. Science 250, 1132-1135.)

### Herstellung von P-Selektin-IgG1

Zur Herstellung des löslichen P-Selektin-IgG1 Fusionsproteins wird das von Aruffo et al., 1991 publizierte genetische Konstrukt "CD62Rg" verwendet. Die weitere Vorgehensweise entspricht der unter A1 dargestellten Herstellung von L-Selektin-IgG1.

Aruffo,A., Kolanus, W., Walz, G., Fredman, P. und Seed, B. 1991. CD62/-P-Selectin recognition of myeloid and tumor cell sulfatides. Cell 67, 35-44.

### Herstellung von CD4-IgG1

Zur Herstellung des löslichen CD4-IgG1 Fusionsproteins wird das von Zettlemeissl et al., 1990 publizierte genetische Konstrukt "CD4:IgG1 hinge" verwendet. Die weitere Vorgehensweise entspricht der unter A1 dargestellten Herstellung von L-Selektin-IgG1. (Zettelmeissl, G., Gregersen, J.-P., Duport, J. M. Mehdi, S., Reiner, G. und Seed, B. 1990. Expression and characterization of human CD4: Immunoglobulin Fusion Proteins. DNA and Cell Biology 9, 347-353.)

### Durchführung des HL60 Zelladhäsionsassays auf rekombinanten, löslichen Adhäsionsmolekülen

1. 96er Mikrotitertestplatten (Nunc Maxisorb) werden mit 100 µl eines in 50 mM Tris pH 9,5 verdünnten (1 + 100) Ziege anti human IgG Antikörpers (Sigma) 2 Std. bei Raumtemperatur inkubiert. Nach Entfernen der Antikörperlösung wird einmal mit PBS gewaschen.
2. 150 µl des Blockierungspuffers werden für 1 Std. bei Raumtemperatur in den Näpfchen belassen. Die Zusammensetzung des Blockierungspuffers ist: 0,1 % Gelatine, 1 % BSA, 5 % Kalbserum, 0,2 mM PMSF, 0,02 % Natriumazid. Nach Entfernen des Blockierungspuffers wird einmal mit PBS gewaschen.
3. In die Näpfchen werden je 100 µl Zellkulturüberstand von entsprechend transfektierten und exprimierten COS-Zellen pipettiert. Die Inkubation erfolgt 2 Std. bei Raumtemperatur. Nach Entfernen des Zellkulturüberstandes wird einmal mit PBS gewaschen.
4. In die Näpfchen werden 20 µl Bindungspuffer gegeben. Der Bindungspuffer hat die Zusammensetzung: 50 mM Hepes, pH 7,5; 100 mM NaCl; 1 mg/ml BSA;
   2 mM MgCl₂; ImMCaCl₂, 3mM MnCl₂; 0,02 % Natriumazid; 0,2 mM PMSF. Dazu werden 5 µl der Testsubstanz pipettiert, durch Schwenken der Platte vermischt und 10 Min. bei Raumtemperatur inkubiert.
5. 50 ml einer HL60 Zellkultur mit 200.000 Zellen/ml werden 4 Min. bei 350 g zentrifugiert. Das Pellet wird in 10 ml RPMI 1640 resuspendiert und die Zellen erneut zentrifugiert. Zur Markierung der Zellen werden 50 µg BCECF-AM (Molecular Probes) in 5 µl wasserfreiem DMSO aufgelöst; anschließend werden 1,5 ml RPMI 1640 auf die BCECF-AM/DMSO-Lösung gegeben. Mit dieser Lösung werden die Zellen resuspendiert und 30 Min. bei 37°C inkubiert. Nach zweiminütiger Zentrifugation bei 350 g wird das markierte Zellpellet in 11 ml Bindungspuffer resuspendiert und die resuspendierten Zellen in 100 µl Aliquots in die Mikrotiterplatten-Näpfchen verteilt. Die Platte wird 10 Min. bei Raumtemperatur stehen gelassen, um die Zellen auf den Boden der Testplatte sedimentieren zu lassen. Dabei haben die Zellen Gelegenheit an das beschichtete Plastik zu adhärieren.
6. Zum Abstoppen des Tests wird die Mikrotiterplatte im 45° Winkel gänzlich in den Stoppuffer getaucht (25 mM Tris, pH 7,5; 125 mM NaCl; 0,1 % BSA; 2 mM MgCl₂; 1 mM CaCl₂; 3 mM MnCl₂; 0,02 % Natriumazid). Durch Invertierung wird der Stoppuffer aus den Näpfchen entfernt und die Prozedur noch zweimal wiederholt.
7. Die Messung der in den Näpfchen festhaftenden, BCECF-AM markierten Zellen erfolgt in einem Cytofluorimeter (Millipore), bei einer Empfindlichkeitseinstellung von 4, einer Anregungswellenlänge von 485/22 nm und einer Emissionswellenlänge von 530/25 nm.

### Ergebnisse:

| IC 50-Werte für P-Selektin und für E-Selektin: | | |
|---|---|---|
| | IC 50 (µM, P-Selectin) | IC 50 (µM, E-Selectin) |
| Polyacrylsäure 995 Na-Salz | 165,00 | > 2000 |
| Polyacrylsäure 2'100 Na-Salz | 125,00 | > 2000 |
| Polyacrylsäure 4'100 Na-Salz | 21,00 | > 2000 |
| Polyacrylsäure 62'900 Na-Salz | 0,009 | > 2000 |
| (Spezifikationen gemäß Angaben der Firma Fluka, Katalog 1996). | | |

### Beispiel 2:

### Leukozyten-Adhäsion - Prüfung der Wirksamkeit der erfindungsgemäßen Verbindungen in vivo (Intravitalmikroskopie an der Ratte):

Bei entzündlichen Prozessen und anderen, die Zytokine aktivierenden Zuständen spielt die Gewebszerstörung durch einwandernde oder die Mikrozirkulation blockierende Leukozyten eine entscheidende Rolle. Die erste und für den weiteren Krankheitsprozeß entscheidende Phase ist die Aktivierung von Leukozyten innerhalb der Blutbahn, insbesondere im prä- und postkapillären Bereich. Dabei kommt es, nachdem die Leukozyten den Axialstrom des Blutes verlassen haben, zu einem ersten Anheften der Leukozyten an der Gefäßinnenwand, d.h. im Gefäßendothel. Alle darauf folgenden Leukozyteneffekte, d.h. die aktive Durchwanderung durch die Gefäßwand und die anschließende orientierte Wanderung im Gewebe, sind Folgereaktionen (Harlan, J.M., Leukocyte-endothelial interaction, Blood 65, 513-525, 1985).

Diese rezeptorvermittelte Interaktion von Leukozyten und Endothelzellen wird als ein initiales Zeichen des Entzündungsprozesses angesehen. Neben den schon physiologisch exprimierten Adhäsionsmolekülen kommt es unter der Einwirkung vom Entzündungsmediatoren (Leukotriene, PAF) und Zytokinen (TNF-alpha, Interleukinen) zur zeitlich gestuften, massiven Expression von Adhäsionsmolekülen auf den Zellen. Sie werden derzeit in drei Gruppen eingeteilt: 1. Immunglobulin-Gensuperfamilie, 2. Integrine und 3. Selektine. Während die Adhäsion zwischen Molekülen der Ig-Gensuperfamilie und den Protein-Protein-Bindungen abläuft, stehen bei der Kooperation zwischen Selektinen Lektin-Kohlehydrat-Bindungen im Vordergrund (Springer, T.A., Adhesion receptors of the immune system. Nature 346, 425-434, 1990; Huges, G., Cell adhesion molecules - the key to an universal panacea, Scrips Magazine 6, 30-33, 1993; Springer, T.A., Traffic signals for lymphocyte recirculation and leukocyte emigration; The multistep paradigm. Cell 76, 301 - 314, 1994).

### Methode:

Die induzierte Adhäsion von Leukozyten wird mit einer intravitalmikroskopischen Untersuchungstechnik im Mesenterium der Ratte quantifiziert (Atherton A. and Born G.V.R., Quantitative investigations of the adhesiveness of circulation polymorphnuclear leukocytes to blood vessel walls. J. Physiol. 222, 447-474, 1972; Seiffge, D. Methoden zur Untersuchung der Rezeptor-vermittelten Interaktion zwischen Leukozyten und Endothelzellen im Entzündungsgeschehen, in: Ersatz- und Ergänzungsmethoden zu Tierversuchen in der biomedizinischen Forschung, Schöffl, H. et al., (Hrsg.) Springer, 1995 (im Druck)). Unter Inhalations-Äthernarkose wird eine Dauernarkose durch intramuskuläre Injektion von Urethan (1,25 mg/kg KG) eingeleitet. Nach Freipräparation von Gefäßen (V. femoralis zur Injektion von Substanzen und A. carotis zur Blutdruckmessung) werden Katheter in diese eingebunden. Danach wird das entsprechende transparente Gewebe (Mesenterium) nach den in der Literatur bekannten Standardmethoden freigelegt und auf dem Mikroskoptisch ausgelagert und mit 37°C warmen Paraffinöl überschichtet (Menger, M.D. and Lehr, H., A. Scope and perspetives of intravital microscopy-bridge over from in vitro to in vivo, Immunology Today 14, 519-522, 1993). Die Applikation der Testsubstanz erfolgt i.v. am Tier (10 mg/kg). Die experimentelle Erhöhung der Blutzell-Adhäsion wird durch systemische Verabreichung von Lipopolysaccharid (LPS, 15 mg/kg) 15 Minuten nach Applikation der Testsubstanz durch Zytokin-Aktivierung ausgelöst (Foster S.J., Mc Cormick L.M., Ntolosi B.A. and Campbell D., Production of TNF-alpha by LPS-stimulated murine, rat and human blood and its pharmacological modulation, Agents and Actions 38, C77-C79, 1993, 18.01.1995). Die dadurch verursachte erhöhte Adhäsion von Leukozyten am Endothel wird direkt vitalmikroskopisch oder mit Hilfe von Fluoreszenzfarbstoffen quantifiziert. Alle Meßvorgänge werden per Videokamera aufgenommen und auf einem Videorekorder gespeichert. Über einen Zeitraum von 60 Minuten wird alle 10 Minuten die Anzahl der rollenden Leukozyten (d.h. alle sichtbar rollenden Leukozyten, die langsamer als die strömenden Erythrozyten sind) und die Anzahl an haftenden Leukozyten am Endothel (Verweildauer länger als 5 Sekunden) erfaßt. Nach Beendigungen des Versuches werden die narkotisierten Tiere schmerzfrei durch systemische Injektion von T61 exzitationsfrei eingeschläfert. Zur Auswertung werden die Ergebnisse jeweils von 8 behandelten mit 8 unbehandelten Tieren (Kontrollgruppe) verglichen (Angabe der Ergebnisse in Prozenten).

### Intravitalmikroskopie an der Ratte:

a) Polyacrylsäure (2'100 Na-Salz): Dosis: 3 mg/kg; Applikation: i.v.; Spezies:
   SPRD (m); Gewicht in g:
      284 +/- 9,6; Anzahl der Gefäße: 15; Gefäßdurchmesser in µm: 28 +/- 2,2; Leukocyten in 10³/mm³: 8,2 +/- 2,; Fibrinogen in mg/100 ml: 96 +/- 10,1; Inhibition: -3 %
b) Polyacrylsäure (62'900 Na-Salz): Dosis: 3 mg/kg; Applikation: i.v.; Spezies:
   SPRD (m); Gewicht in g:
      276 +/- -13,6; Anzahl der Gefäße: 18; Gefäßdurchmesser in µm: 27 +/- 5,2; Leukocyten in 10³/mm³: 12,5 +/- 3,5; Fibrinogen in mg/100 ml: 101 +/- 12,3; Inhibition: 64 %.

## Patentansprüche

1. Polymer der Acrylsäure oder Methacrylsäure oder deren pharmakologisch verträglichen Salze zur Anwendung als Arzneimittel.

2. Polymer nach Anspruch 1, dadurch gekennzeichnet, daß das Polymer eine Polyacrylsäure ist.

3. Polymer nach Anspruch 2, dadurch gekennzeichnet, daß das Polymer das Natriumsalz einer Polyacrylsäure ist.

4. Polymer nach einem der Ansprüche 1 bis 3, dadurch gekennzeichnet, daß die mittlere Molmasse des Polymers 900 bis 63 000 g/mol beträgt.

5. Verwendung eines Polymers gemäß einem der Ansprüche 1 bis 4 zur Herstellung eines Arzneimittels zur Therapie oder Prophylaxe einer Erkrankung, die mit einer übermäßigen, durch Selektinrezeptoren vermittelten Zelladhäsion in dem von der Krankheit betroffenen Gewebe einhergeht.

6. Verwendung nach Anspruch 5, dadurch gekennzeichnet, daß die Krankheit eine Herz-Kreislauf-Erkrankung ist.

7. Verwendung nach Anspruch 5, dadurch gekennzeichnet, daß die Krankheit eine rheumatische Erkrankung ist.

8. Arzneimittel enthaltend mindestens ein Polymer gemäß einem der Ansprüche 1 bis 4 und pharmazeutische Hilfsstoffe.
